(19) Europäisches Patentamt
European Patent Office
Office européen des brevets

(11) Publication number: **0 334 967 B1**

# (12) EUROPEAN PATENT SPECIFICATION

(45) Date of publication of patent specification: **11.08.93**

(51) Int. Cl.$^5$: **A61K 31/12**, C07C 49/255

(21) Application number: **88908350.7**

(22) Date of filing: **22.09.88**

(86) International application number:
**PCT/JP88/00959**

(87) International publication number:
**WO 89/03376 (20.04.89 89/09)**

## (54) KERATOSIS-TREATING AGENT.

(30) Priority: **06.10.87 JP 250776/87**
**12.02.88 JP 28721/88**

(43) Date of publication of application:
**04.10.89 Bulletin 89/40**

(45) Publication of the grant of the patent:
**11.08.93 Bulletin 93/32**

(84) Designated Contracting States:
**AT BE CH DE FR GB IT LI NL SE**

(56) References cited:
**JP-A- 5 129 401**
**JP-A- 5 746 914**
**JP-A- 6 372 625**
**JP-A-61 137 834**
**JP-A- 5 629 540**
**JP-A- 6 084 231**
**JP-A-61 134 338**
**JP-A-61 191 625**

**PROSTAGLANDINS, LEUKOTRIENES AND MEDICINE, vol. 24, nos. 2/3, October 1986, pages 195-198; D.L. FLYNN et al.: "Inhibition of human neutrophil 5-lipoxygenase activity by gingerdione, shogaol, capsaicin and related pungent compounds"**

(73) Proprietor: **TSUMURA & CO.**
**4-10, Nihonbashi 3-chome**
**Chuo-ku, Tokyo 103(JP)**

(72) Inventor: **UDA, Katsuya**
**Tsumura Juntendo, Inc. 3586, Yoshiwara**
**Ami-machi Inashiki-gun Ibaragi 300-11(JP)**
Inventor: **SAKAMOTO, Kenji**
**Tsumura Juntendo, Inc. 3586, Yoshiwara**
**Ami-machi Inashiki-gun Ibaragi 300-11(JP)**
Inventor: **SUEKAWA, Mamoru**
**Tsumura Juntendo, Inc. 3586, Yoshiwara**
**Ami-machi Inashiki-gun Ibaragi 300-11(JP)**
Inventor: **YAMAMOTO, Takeshi**
**Tsumura Juntendo, Inc. 3586, Yoshiwara**
**Ami-machi Inashiki-gun Ibaragi 300-11(JP)**
Inventor: **OHTA, Yuichiro**
**Tsumura Juntendo, Inc. 3586, Yoshiwara**
**Ami-machi Inashiki-gun Ibaragi 300-11(JP)**
Inventor: **CHISAKI, Keigo**
**Tsumura Juntendo, Inc. 3586, Yoshiwara**
**Ami-machi Inashiki-gun Ibaragi 300-11(JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3. 10/3.6/3.3. 1)

**CHEMICAL ABSTRACTS, vol. 106, no. 12, 23rd March 1987, page 349, abstract no. 89987e, Columbus, Ohio, US; & JP-A-61 263 909 (SHISEIDO CO., LTD) 21-11-1986**

**JOURNAL OF CHROMATOGRAPHY, vol. 360, 1986, pages 175-184, Elsevier Science Publishers B.V., Amsterdam, NL; C.-C. CHEN et al.: "Chromatographic analyses of isomeric shogaol compounds derived from isolated gingerol compounds of ginger (Zingiber officinale Roscoe)"**

Inventor: **ABURADA, Masaki**
**Tsumura Juntendo, Inc. 12-7, Niban-cho**
**Chiyoda-ku Tokyo 102(JP)**

(74) Representative: **Stuart, Ian Alexander et al**
**MEWBURN ELLIS & CO. 2/3 Cursitor Street**
**London EC4A 1BO (GB)**

## Description

The present invention relates to the use of 6-schogaols in the manufacture of an agent for the remedy of keratodermia; and to a novel process for preparing cis-(6)-shogaol.

Zingeron, trans-(6)-shogaol and gingerol are known as components contained in crude drugs, such as Zinqiberis siccatum rhizoma and Zinqiberis rhizoma, which are incorporated in Chinese medicine preparations such as Chai-Hu-Gui-Zhi-Gan-Jian-Tang, Xiao-Qing-Long-Tang, and Ge-Gen-Tang, and it has been confirmed that trans-(6)-shogaol has an antipyretic action, an analgetic action, and an antiepileptic action.

Recently, there has been an increase in the occurrence keratodermia represented by scabies, and as main morphological changes of the skin in a focus portion in scabies, there can be mentioned a thickening of the epidermal cell layer and abnormal keratinization based on a turn-over of highly accelerated epidermal cells, an inflammatory reaction of the epidermal nipple layer, and a straying of multinucleate leucocytes to the epidermal cell layer.

As an agent for the remedy of keratodermia, steroids have been used, but they are disadvantageous in that they have serious side effects, and the use of a vitamin A derivative (retinoid) is restricted because of teratogenicity. Accordingly, an epoch-making agent for the remedy of keratodermia has not been found.

The present inventors carried out research into the development of a medicine effective for the remedy of keratodermia, and as a result, found that (6)-shogaol has an excellent remedial effect on keratodermia, and the present invention was completed based on this finding.

More specifically, in accordance with the present invention, there is provided the use for the manufacture of an agent for the remedy of keratodermia (hereinafter referred to as "medicine of the present invention") of at least one compound selected from trans-(6)-shogaol [1(4-hydroxy-3-methoxyphenyl)-(E)-4-decen-3-one] represented by the following formula:

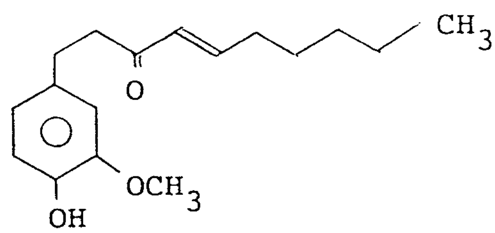

and cis-(6)-shogaol represented by the following formula:

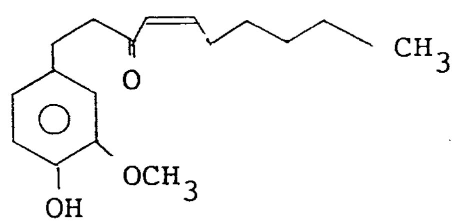

Trans-(6)-shogaol, which maybe the effective ingredient of a medicine of the present invention, is contained in crude drugs, such as Zinqiberis siccatum thizoma and Zinqiberis rhizoma, which are incorporated in Chinese medicine preparations such as Chai-Hu-Gui-Zhi-Gan-Jian-Tang, Xiao-Qing-Long-Tang, and Ge-Gen-Tang, and it is known that this compound has a nerve center-controlling action and an analgetic action.

This trans-(6)-shogaol is prepared, for example, in the following manner.

A dry rootstock of a ginger is extracted with an organic solvent such as ether, ethanol or methanol, and the obtained extract is subjected to column chromatography using a silica gel as the carrier, and the development is carried out by using an organic solvent such as n-hexane, acetone, benzene, ethyl acetate or ether or a mixed solvent thereof, to obtain a fraction containing trans-(6)-shogaol. This fraction is refined by preparative thin-layer chromatography.

A specific example of the preparation of trans-(6)-shogaol will now he described.

Specific Example

A dry rootstock (2 kg) of a ginger (Zinqiber officinale roscoe) was extracted under reflux with 3 ℓ of ether for 7 hours, and the residue was further extracted under reflux with 3 ℓ of ether twice for 7 hours. The extract was combined, and was concentrated under a reduced pressure to obtain 77.05 g of an extract.

Then, 77.05 g of the ether extract was subjected to column chromatography using 700 g of a silica gel (Kieselgel supplied by Merck), and eluting with a mixed solvent of n-hexane and ether while gradually increasing the ratio of ether.

A fraction eluted with 0.6 ℓ of n-hexane/ether (70/30) was combined with a fraction eluted with 1.6 ℓ of n-hexane/ether (60/40), and the solvents were removed to obtain 13.6 g of a residue. Then, the residue was again subjected to column chromatography using the silica gel, a fraction eluted with n-hexane/ether (95/5) was collected, and the solvents were removed to obtain 5.48 g of a residue. The residue was subjected to preparative thin-layer chromatography [plate = Kieselgel 60 PF254; eluents = n-hexane/acetone (7/3)] to obtain 803 mg (yield = 0.04%) of a light-yellow oily substance. The physical and chemical properties of this light-yellow oily substance were in agreement with those of trans-6-shogaol disclosed in the pertinent references.

Cis-(6)-shogaol can be obtained by irradiating this trans-(6)-shogaol with ultraviolet rays. More specifically, cis-(6)-shogaol can be obtained by dissolving trans-(6)-shogaol obtained in the above-mentioned manner in an organic solvent such as methanol or ethanol and irradiating the solution with ultraviolet rays of a low-pressure mercury lamp for 1 to 3 hours.

Cis-(6)-shogaol has not previously been isolated preparatively, though Chen et.al,J.Chromatog. 360-(1986) 175-184, disclose a mixture which apparently contained cis-6-shogaol, and which was separated by analytical HPLC.

With reference to the following experiments, it will now be proved that trans-(6)shogaol has a curative effect on keratodermia.

Experiment 1

Inhibitory action to EGF stimulated cell qrowth

A culture medium (hereinafter referred to as "CS DMEM") containing 10% of calf serum, which was prepared so that 2.5 x $10^4$ of fibroblasts (balb/c 3T3 cells) were contained in 0.5 ml, was added in a 24-well plate in an amount of 0.5 ml per well. After the fibroblasts were confluent in the state of a single layer, the culture medium was replaced with the same amount of 1% CS DMEM and the cells were incubated at 37°C for 12 hours in a humidified atmosphere of 95% air-5% $CO_2$. Then, 1 $\mu$g/25 $\mu$l/well of trans-(6)-shogaol and 5 ng/25 $\mu$l/well of EGF (supplied by Collaborative Research, Inc.) were added, and the cells were further incubated for 16 hours. Then, 0.5 $\mu$Ci($1.85 + 10^{-16} S^{-1}$)/50 $\mu$l of $^3$H-thymidine was added and the cells were further incubated 8 hours. The culture medium was removed, and the cells were washed with a cold phosphate buffered saline. Then, 5% cold trichloroacetic acid was added and the cells were allowed to stand for 30 minutes. The supernatant was removed, the cells were dissolved in a 0.5M aqueous solution of sodium hydroxide, and the solution was neutralized with 0.5M hydrochloric acid. Scintillation cocktail was added to the liquid and counting was carried out by a liquid scintillation counter. The radioactivity of $^3$H-thymidine included in the cells by this growth was measured, and the action of trans-(6)-shogaol to the growth of the cells was examined. It was found that 52.7% of the growth was inhibited to balb/c 3T3 cells, the growth of which was promoted by EGF.

From this result, it was confirmed that trans-(6)-shogaol has a curative effect on keratodermia.

When the acute toxicity test of trans-(6)-shogaol was carried out by using male mice of the ICR line, it was found that the $LD_{50}$ was 50.9 mg/kg (up and down method) for an intravenous administration and 687 mg/kg (Litchfield-Wilcoxon method) for an oral administration.

As apparent from this result, trans-(6)-shogaol has a low toxicity and is very safe.

With reference to the following experiments, it will now be proved that cis-(6)=shogaol has a 5-lipoxygenase-inhibiting action, a cyclooxygenase-inhibiting action, and a curative effect on keratodermia.

Experiment 2

5-Lipoxyqenase-Inhibitinq Action

RBL-1 cultured cells were suspended in a 50 mM phosphate buffer solution (pH 7.4) containing 1 mM EDTA and 10% ethylene glycol so that the cell concentration was 5 x $10^6$ per ml, and the cell suspension was subjected to an ultrasonic wave treatment. Then, centrifugal separation was carried out under 10,000 x G for 10 minutes and then under 105,000 x G for 60 minutes. The supernatant was used as the standard enzyme of 5-lipoxygenase.

A test tube was charged with a 50 mM phosphate buffer solution, 1.5 mM calcium chloride, 20 µM indomethacin, 18 mM glutathione, 6 mM ATP, 10 µM arachidonic acid, the obtained standard enzyme, and an ethanol solution of cis-(6)-shogaol, to a total volume of 0.6 ml and a final concentration of 5, 1 or 0.5 µM, and a reaction was carried out at 37°C for 10 minutes. After termination of the reaction, 1.2 ml of acetone and 100 µl of 2N formic acid were added to stop the reaction, and 10 µl of n-butyl-3,5-dinitrobenzoate was added as the internal standard. The mixture was extracted with 1.8 ml of n-hexane. The obtained extract was concentrated and dissolved in methanol. The amount of 5-HETE in the liquid measured by the high-speed liquid chromatography [column = TSK-gel ODS-80TM (supplied by Toso), moving phase = acetonitrile/water/ acetic acid (60/40/0.02), flow rate = 1 ml/min, detection = ultraviolet ray (235 nm)], and the obtained value was designated as the enzyme activity. The inhibition ratio at each concentration was calculated by the following formula, and the concentration giving an inhibition ratio of 50% (hereinafter referred to as "$IC_{50}$") was determined.

$$\text{Inhibition ratio} = \frac{C - S}{C} \times 100\ (\%)$$

wherein C stands for the peak area of 5-HETE (corrected by the internal standard) obtained when cis-(6)-shogaol was not incorporated, and S stands for the peak area of 5-HETE (corrected by the internal standard) obtained when cis-(6)-shogaol was added.

As a result, it was found that the $IC_{50}$ of cis-(6)-shogaol was 0.83 µM.

Experiment 3

Cyclooxygenase-inhibiting action

To 7.0 g of rabbit kidney marrow was added 60 ml of a 0.1M phosphate buffer solution (pH 7.5) containing 1 mM glutathione, and the mixture was homogenized by using a polytrone. The homogenate was subjected to the centrifugal separation under. 105,000 x G for 60 minutes to obtain a microsome fraction, and the fraction was dissolved in 7 ml of a 0.1M phosphate buffer solution (pH 7.5) containing 1 mM glutathione to obtain a standard enzyme of cyclooxygenase.

To 15 µl of the obtained standard enzyme were added 4 mM glutathione, 10 mM tryptophan, 0.25 µM hemoglobin, and a phosphate buffer solution (pH 7.5), each concentration being the final concentration, and [1-$^{14}$C] arachidonic acid (5 x $10^4$ dpm) and cis-(6)-shogaol were added to a final concentration of 40, 20, 10 or 5 µM and a total volume of 200 µl. Incubation was carried out at 37°C for 15 minutes, and 1N hydrochloric acid was added to stop the reaction. Prostaglandin $E_2$ ($PGE_2$) was added as the carrier and the reaction product was extracted with 2 ml of ether. The obtained extract was concentrated and subjected to thin layer chromatography [developing solvent = chloroform/methanol/acetic acid (18/1/1)] to separate the product. Coloration was effected with an iodine vapor, the portion corresponding of $PGF_2$ was scratched out, and the radioactivity of $PGE_2$ was measured by a liquid scintillation counter. The inhibition ratio at each concentration was calculated according to the following formula, and the $1C_{50}$ was determined.

$$\text{Inhibition ratio} = \frac{C - S}{C} \times 100\ (\%)$$

wherein C stands for the amount formed of $PGE_2$ observed when cis-(6)-shogaol was not added, and S stands for the amount formed of $PGE_2$ observed when cis-(6)-shogaol was added.

As the result, it was found that the $IC_{50}$ of cis-(6)-shogaol was 11.7.

Experiment 4

Inhibitory action to EGF stimulated cell growth

10% CS DMEM prepared so that 2.5 x $10^4$ of fibroblasts (balb/c 3T3 cells) were contained in 0.5 ml were added in a 24-well plate in an amount of 0.5 ml/well. After the fibroblasts were confluent in the state of a single layer, the medium was replaced with the same amount of 1% CS DMEM, and the cells were incubated at 37°C for 12 hours in a humidified atmosphere of 95% of air-5% $C0_2$. Then, 0.5 $\mu$g/12.5 $\mu$l/well of cis-(6)-shogaol and 50 pg/25 $\mu$l/well of mouse EGF were added and the cells were further incubated for 16 hours. Then, 0.5 $\mu$Ci($1.85x10^{-16}$.$S^{-1}$)/50 $\mu$l/well of $^3$H-thymidine was added and the cells were further incubated for 8 hours. The culture medium was removed, and the cells were washed with a cold isotonic phosphate buffered saline and 5% cold trichloroacetic was added. Then, the cells were allowed to stand for 30 minutes. The supernatant was removed, and the cells were dissolved in a 0.5M aqueous solution of sodium hydroxide and neutralized with 0.5M hydrochloric acid. Scintillation cocktail was added and counting was carried out by a liquid scintillation counter. The radioactivity of $^3$H-thymidine included in the cells by this growth was measured, and the function of cis-(6)-shogaol to the growth of the cells was examined. As a result, it was found that 63.9% of the growth was inhibited to balb/c 3T3 cells, the growth of which was promoted by EGF.

From the foregoing result, it was found that cis-(6)-shogaol has an excellent curative effect on keratodermia.

The acute toxicity test of cis-(6)-shogaol was carried out by using male mice of the ICR line. It was found that none of the mice died at an oral administration of 500 mg/kg.

Thus, it was confirmed that cis-(6)-shogaol has a low toxicity and is very safe.

The dose and preparation of (6)-shogaol [cis- or trans-(6)-shogaol; the same will apply hereinafter], which is the effective ingredient of the present invention, will now be described.

(6)-Shogaol can be administered to a man or animal singly or together with a customary pharmaceutical carrier. The administration form is not particularly critical, and an appropriate administration form can be selected and used. For example, there can be mentioned medicines for oral administration, such as a tablet, a capsule, a granule, a fine particle and a powder, and medicines for non-oral administration, such as an injection and a suppository.

To obtain an intended effect by an oral administration, 20 to 80 mg of (6)-shogaol is usually administered per day in several separate doses for an adult, although the daily dosage differs according to the age and body weight of a patient and the degree of the disease.

In the present invention, a medicine for oral administration, such as a tablet, a capsule or a granule, can be prepared according to customary procedures by using, for example, starch, lactose, refined sugar, mannitol, carboxymethyl cellulose, corn starch, and an inorganic salt.

Not only the above-mentioned excipients but also hinders, disintegrating agents, surface active agents, lubricants, flowability improvers, taste improvers, colorants and perfumes can be incorporated into preparation of this type. Specific examples of these additives are described below.

(Binders)

Starch, dextrin, gum arabic powder, gelatin, hydroxypropylstarch, methylcellulose, sodium carboxymethylcellulose, hydroxypropylcellulose, crystalline cellulose, ethylcellulose, polyvinyl pyrrolidone and macrogol.

(Disintegrating Agents)

Starch, hydroxypropylstarch, sodium carboxymethyl-cellulose, calcium carboxymethylcellulose and lowly substituted propylcellulose.

(Surface Active Agents)

Sodium lauryl sulfate, soybean lecithin, sucrose fatty acid ester and polysolvate 80.

(Lubricants)

Talc, wax, hydrogenated vegetable oil, sucrose fatty acid ester, magnesium stearate, calcium stearate, aluminum stearate and polyethylene glycol.

(Flowability Improvers)

Soft silica, dried aluminum hydroxide gel, synthetic aluminum silicate and magnesium silicate.

The medicine of the present invention can be administered in a suspension, an emulsion, a syrup or an elixir. In preparations of this type, a taste improver, a smell improver and a colorant can be incorporated.

To obtain an intended effect for a medicine for non-oral administration, (6)-shogaol is usually administrated at a dosage of 0.25 to 10 mg per day for an adult by intravenous injection, intravenous drip, hypodermic injection or intramuscular injection, although the daily dosage differs according to the age and body weight of a patient and the degree of the disease.

These medicines for non-oral administration can be prepared according to customary procedures by using diluents such as distilled water for injection, physiological saline solution, an aqueous solution of glucose, a vegetable oil for injection, rubber oil, peanut oil, soybean oil, corn oil, propylene glycol and polyethylene glycol. A fungicide, an antiseptic agent and a stabilizer can be added according to need. In view of the stability of the medicine for non-oral administration, a method can be adopted in which the medicine is filled and frozen in a vial, water is removed by a customary freeze-drying technique, and a liquid preparation is prepared from the freeze-dried product just before administration. An isotropic agent, a stabilizer, an antiseptic agent and an indolent agent can be added according to need.

An external liquid preparation, a coating agent such as an ointment, and a suppository for the rectal administration can be mentioned as other non-oral preparations, and are prepared according to customary procedures.

The present invention will now be described in detail with reference to the following examples

Example 1

In 1 ml of methanol was dissolved 29 mg of trans-(6)-shogaol prepared in the specific example given hereinbefore, and the solution was charged in a quartz cell and irradiated for 1 hour with ultraviolet rays from a low-pressure mercury lamp. After the reaction, the reaction mixture was subjected to the column chromatography (ether/hexane = 2/8) using a silica gel in an amount 30 times the amount of the reaction mixture. Fractions, each being 5 ml, were collected, and 18th through 22nd fractions were combined to obtain 7.5 mg of cis-(6)-shogaol in the form of a light-yellow oily substance having the following physical and chemical properties. Infrared absorption spectrum

$$\nu \frac{KBr}{max} cm^{-1}:$$

3540, 1680

Proton nuclear magnetic resonance spectrum (δ ppm in $CDC1_3$):

0.89 (3H, t, J = 7.0 Hz) , 1.31 (4H, m), 1.42 (2H, m), 2.61 (2H, dt, J = 7.0, 6.9 Hz), 2.75 (2H, t, J = 7.5 Hz), 2.86 (2H, t, J = 7.5 Hz), 3.87 (3H, s), 6.07 (1H, dt, J = 7.0, 11.5 Hz), 6.12 (1H, dt, J = 1.3, 11.5 Hz) , 6.68 (1H, dd, J = 1.9, 8.0 Hz), 6.70 (1H, d, J = 1.9 Hz), 6.82 (1H, d, J = 8.0 Hz)

$^{13}C$-Nuclear magnetic resonance spectrum (δ ppm in $CDC1_3$):

14.0, 22.5, 28.9, 29.5, 29.7, 31.5, 46.1, 55.9, 111.1, 114.3', 120.9, 126.6, 133.2, 143.9, 146.4, 149.0

Mass spectrum:

m/z (%) 276 ($M^+$, 27)

205 (27),

137 (100), 55 (37)

Example 2

| | | |
|---|---|---|
| (1) | Corn starch | 23.5 g |
| (2) | Crystalline cellulose | 15 g |
| (3) | Calcium carboxymethylcellulose | 5 g |
| (4) | Light silica | 0.5 g |
| (5) | Magnesium stearate | 1 g |
| (6) | (6)-Shogaol | 5 g |
| | Total | 50 g |

Components (1) through (6) were homogeneously mixed according to the above-mentioned recipe, and the mixture was compression-molded by a tablet machine to obtain tablets, each weighing 200 mg.

Each table contained 20 mg of (6)-shogaol, and 1 to 4 tablets were administered per day, separately at different times, for an adult.

Example 3

| | | |
|---|---|---|
| (1) | Crystalline cellulose | 39.5 g |
| (2) | Magnesium stearate | 0.5 g |
| (3) | Calcium carboxymethylcellulose | 5 g |
| (4) | (6)-Shogaol | 5 g |
| | Total | 50 g |

According to the above-mentioned recipe, components (1) and (4) and a part of component (2) were homogeneously mixed, and the mixture was compression-molded and pulverized. Component (3) and the remainder of component (2) were added to the pulverization product, and the mixture was compression-molded by a table machine to obtain tablets, each weighing 200 mg.

Each tablet contained 20 mg of (6)-shogaol, and 1 to 4 tablets were administered per day, separately at different times, for an adult.

Example 4

| | | |
|---|---|---|
| (1) | Crystalline cellulose | 17 g |
| (2) | 10% Ethanol solution of hydroxy-propylcellulose | 25 g |
| (3) | Calcium carboxymethylcellulose | 2.7 g |
| (4) | Magnesium stearate | 0.3 g |
| (5) | (6)-Shogaol | 5 g |
| | Total | 50 g |

According to the above-mentioned recipe, components (1), (2) and (5) were homogeneously mixed, and according to customary procedures, the mixture was kneaded and granulated by an extrusion granulator. The granulation product was dried and disintegrated and was mixed with components (3) and (4). The mixture was compression-molded by a tablet machine to obtain tablets, each weighing 200 mg.

Each tablet contained 20 mg of (6)-shogaol, and 1 to 4 tablets were administered per day, separately at different times, for an adult.

Example 5

| | | |
|---|---|---|
| (1) | Corn starch | 43 g |
| (2) | Magnesium stearate | 0.5 g |
| (3) | Calcium carboxymethylcellulose | 5 g |
| (4) | Soft silica | 0.5 g |
| (5) | (6)-Shogaol | 1 g |
| | Total | 50 g |

According to the above-mentioned recipe, components (1) through (5) were homogeneously mixed, and the mixture was compression-molded by a compression molding machine. The molded body was pulverized by a pulverizer and classified to obtain a granule.

This granule (1 g) contained 20 mg of (6)-shogaol, and 1 to 4 g of the granule was administered per day, separately at different times, for an adult.

Example 6

| | | |
|---|---|---|
| (1) | Crystalline cellulose | 29 g |
| (2) | 10% Ethanol solution of hydroxy-propylcellulose | 20 g |
| (3) | (6)-Shogaol | 1 g |
| | Total | 50 g |

According to the above-mentioned recipe, components (1) through (3) were mixed and kneaded, and the mixture was granulated by an extrusion extruder, dried and sieved to obtain a granule.

This granule (1 g) contained 20 mg of (6)-shogaol, and 1 to 4 g of the granule was administered per day, separately at different times, for an adult.

Example 7

| | | |
|---|---|---|
| (1) | Corn starch | 44.5 g |
| (2) | Light silica | 0.5 g |
| (3) | (6)Shogaol | 5 g |
| | Total | 50 g |

According to the above-mentioned recipe, components (1) through (3) were homogeneously mixed, and 200 mg of the mixture was filled in a capsule No. 2.

One capsule contained 20 mg of (6)shogaol, and 1 to 4 capsules were administered per day, separately at different times, for an adult.

Example 8

| | | |
|---|---|---|
| (1) | Distilled water for injection | appropriate amount |
| (2) | Glucose | 200 mg |
| (3) | (6)-Shogaol | 1 mg |
| | Total | 5 mg |

Components (2) and (3) were dissolved in distilled water for injection, and the solution was filled in a 5-ml ampoule and subjected to sterilization under pressure at 121 °C for 15 minutes to obtain an injection.

Example 9

| | | |
|---|---|---|
| (1) | (6)-Shogaol | 0.05 g |
| (2) | White vaseline | 25 g |
| (3) | Stearyl alcohol | 22 g |
| (4) | Bleached bees wax | 15 g |
| (5) | Polyoxyethylene(25) monostearate | 2.3 g |
| (6) | Sorbitol monopalmitate | 2.7 g |
| (7) | Propyl p-hydroxybenzoate | 0.05 g |
| (8) | Methyl p-hydroxybenzoate | 0.05 g |
| (9) | Refined water | 32.85 g |
| | Total | 100 g |

According to the above-mentioned recipe, components (1) through (9) were homogeneously mixed and the mixture was heated and melted to obtain an ointment.

**Claims**

1. Use for the manufacture of an agent for the remedy of keratodermia, of at least one compound selected from trans-(6)-shogaol represented by the following formula:-

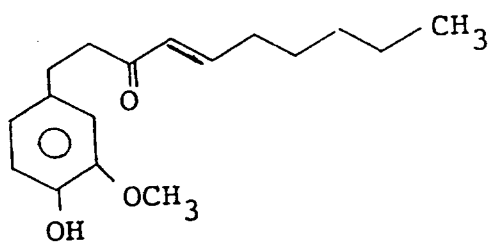

and cis-(6)-shogaol represented by the following formula:

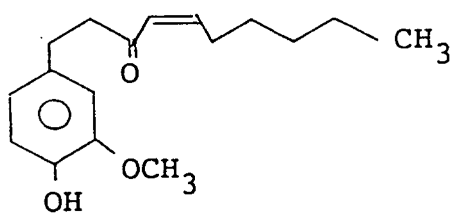

2. Use as set forth in claim 1, wherein the agent is in the form of a preparation for oral administration, and which is selected from among a tablet, a granule, a fine particle, and a powder.

3. Use as set forth in claim 2, wherein the agent is adapted to be administered to an adult at a dose of 20 to 80 mg as the effective ingredient per day.

4. Use as set forth in claim 1, wherein the agent is in the form of a preparation for non-oral administration by intravenous injection, intravenous drip, hypodermic injection, or intramuscular injection.

5. Use as set forth in claim 4, wherein the agent is adapted to be administered to an adult in an amount of 0.25 to 10 mg as the effective ingredient per day.

6. Use as set forth in claim 1, wherein the agent is in the form of a liquid for exterior application, an ointment or a suppository.

7. A method of preparing cis-6-shogaol comprising UV-irradiation of trans-6-shogaol.

**Patentansprüche**

1. Verwendung wenigstens einer Verbindung, ausgewählt aus trans-(6)-Shogaol der folgenden Formel

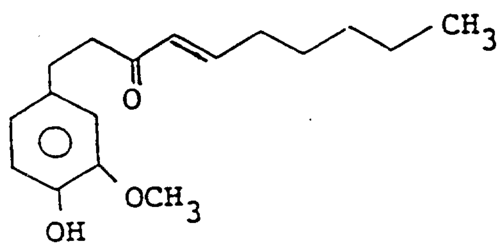

und cis-(6)-Shogaol der folgenden Formel

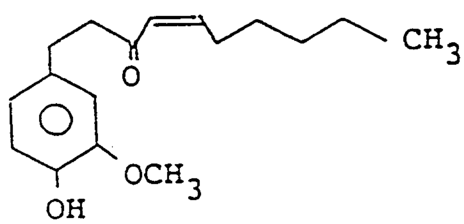

zur Herstellung eines Arzneimittels gegen Keratose.

2. Verwendung nach Anspruch 1, worin das Mittel in Form einer Zubereitung zur oralen Verabreichung als Tablette, Granulat, Pulver und Puder vorliegt.

3. Verwendung nach Anspruch 2, worin das Mittel eingestellt ist, um an einen Erwachsenen in einer Tagesdosis von 20 bis 80 mg des wirksamen Bestandteils verabreicht zu werden.

4. Verwendung nach Anspruch 1, worin das Mittel in Form einer Zubereitung zur nichtoralen Verabreichung durch intravenöse Injektion, intravenöse Infusion, subkutane Injektion oder intramuskulären Injektion vorliegt.

5. Verwendung nach Anspruch 4, worin das Mittel angepaßt ist, an einen Erwachsenen in einer Menge von 0,25 bis 10 mg des wirksamen Bestandteils als Tagesdosis verabreicht zu werden.

6. Verwendung nach Anspruch 1, worin das Mittel in Form einer Flüssigkeit zur äußeren Anwendung als Salbe oder als Suppositorium vorliegt.

7. Verfahren zur Herstellung von cis-6-Shogaol, enthaltend die UV-Bestrahlung von trans-6-Shogaol.

**Revendications**

1. Utilisation pour la fabrication d'un agent pour le traitement de la kératose, d'au moins un composé sélectionné parmi le trans-(6)-shogaol représenté par la formule suivante:

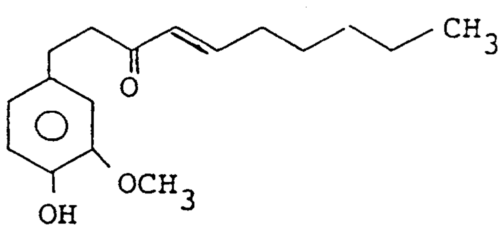

et le cis-(6)-shogaol représenté par la formule:

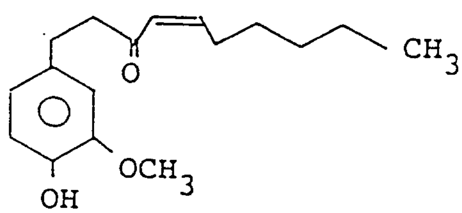

2. L'utilisation selon la revendication 1, caractérisée en ce que l'agent est sous la forme d'une préparation destinée à une administration par voie orale et qui est sélectionné parmi les comprimés, les granules, les particules fines et les poudres.

3. L'utilisation selon la revendication 2, caractérisée en ce que l'agent est ajusté pour être administré à un adulte à une dose quotidienne comprise entre 20 et 80 mg de principe actif.

4. L'utilisation selon la revendication 1, caractérisée en ce que l'agent est sous la forme d'une préparation destinée à une administration par voie parentérale par injection intraveineuse, perfusion intraveineuse, injection hypodermique ou injection intramusculaire.

5. L'utilisation selon la revendication 4, caractérisée en ce que l'agent est ajusté pour être administré à un adulte en une dose quotidienne comprise entre 0,25 et 10 mg de principe actif.

6. L'utilisation selon la revendication 1, caractérisée en ce que l'agent est sous la forme d'une application liquide externe, d'un onguent ou d'un suppositoire.

7. Une méthode de préparation du cis-6-shogaol comprenant l'irradiation aux U.V. du trans-6-shogaol.